Europäisches Patentamt

European Patent Office

Office européen des brevets

(11) Veröffentlichungsnummer: **0 262 571 B1**

(12) **EUROPÄISCHE PATENTSCHRIFT**

(45) Veröffentlichungstag der Patentschrift: **09.12.92**

(21) Anmeldenummer: **87113896.2**

(22) Anmeldetag: **23.09.87**

(51) Int. Cl.⁵: **C12N 5/00**, C12P 21/00, C12N 15/00, C07K 3/18, C07K 15/26, C07K 17/00, G01N 33/577, G01N 33/68, //(C12P21/00,C12R1:91)

(54) **Neue monoklonale Antikörper gegen IFN-omega, Verfahren zu ihrer Herstellung und deren Verwendung zur Reinigung sowie zum Nachweis von IFN-omega.**

(30) Priorität: **01.10.86 DE 3633323**

(43) Veröffentlichungstag der Anmeldung:
**06.04.88 Patentblatt 88/14**

(45) Bekanntmachung des Hinweises auf die Patenterteilung:
**09.12.92 Patentblatt 92/50**

(84) Benannte Vertragsstaaten:
**AT BE CH DE ES FR GB GR IT LI LU NL SE**

(56) Entgegenhaltungen:
**EP-A- 0 170 204**
**EP-A- 0 236 920**
**DE-A- 3 306 060**

(73) Patentinhaber: **BOEHRINGER INGELHEIM INTERNATIONAL GmbH**
**Postfach 20**
**W-6507 Ingelheim am Rhein(DE)**

(72) Erfinder: **Adolf, Günther, Dr.**
**Johannagasse 20/7**
**A-1050 Wien(AT)**

EP 0 262 571 B1

**Beschreibung**

In der EP-A-0 170 204 und in Nucleic Acids Res. 13, 4739-4749 (1985) werden neue Typ I-Interferone beschrieben, die sich in ihrer Struktur und ihren antigenen Eigenschaften wesentlich von den bisher bekannten $\alpha$- und $\beta$-Interferonen unterscheiden. Diese neue Interferon-Klasse wurde daher mit IFN-omega bezeichnet.

Außerdem werden in der DE-A-3 306 060 monoklonale Antikörper, die die Aktivität von Humaninterferon des Typs $\alpha$ ganz oder teilweise neutralisieren, beschrieben.

Desweiteren konnte mit Hilfe der in der EP-A-0.236.920, welche am 16. September 1987 veröffentlicht wurde, beschriebenen neuen monoklonalen Antikörpern, z.B. des neuen monoklonalen Antikörpers OMG-2, die Reinigung der IFN-omega1 wesentlich verbessert werden. Diese Antikörper zeigen jedoch Spezifität sowohl für IFN-$\alpha$ als auch für IFN-omega1. Mit Hilfe der in dieser Anmeldung beschriebenen Antikörper konnte jedoch kein Immunoassay zum Nachweis von IFN-omega1 etabliert werden, da der Anteil der IFN-omega1-spezifischen Antikörper im polyklonalen Immunoglobulin, das zur Beschichtung eingesetzt wurde, viel zu gering war. Darüber hinaus würde ein derartiger Test nicht spezifisch für IFN-omega sein, da, wie bereits oben erwähnt, sowohl die polyklonalen als auch die monoklonalen Antikörper auch IFN-$\alpha$ erkennen würden.

Der Nachweis und die Quantifizierung von IFN-omega mußte daher bisher ausschließlich mit Hilfe von biologischen Tests durchgeführt werden, wie zum Beispiel der Messung der antiviralen Aktivität. Diese Nachweismethoden sind im allgemeinen sehr empfindlich, jedoch zeit- und arbeitsaufwendig sowie von geringer Präzision. Die Etablierung eines Immunoassays, zum Beispiel eines Tests vom Typ eines ELISA oder IRMA, mit dessen Hilfe eine einfache, rasche und genaue Bestimmung von IFN-omega möglich ist, wäre daher wünschenswert. Da IFN-omega in Lösung als Monomeres vorliegt, sind zur Etablierung eines derartigen Tests aber mindestens zwei Antikörper notwendig, die verschiedene Epitope des IFN-Moleküls erkennen können, wobei monoklonale Antikörper zwar nicht notwendig sind, jedoch zahlreiche Vorteile gegenüber Antiseren haben.

Überraschenderweise wurde nun gefunden, daß die erwähnten Probleme mit Hilfe neuer monoklonaler Antikörper behoben werden können. Die erfindungsgemäß hergestellten Antikörper sind spezifisch für IFN-omega; mit ihrer Hilfe hergestellte Immunoassays erlauben den Nachweis und die Quantifizierung von IFN-omega, sprechen jedoch auf andere humane Interferone, wie IFN-$\alpha$, IFN-$\beta$ oder IFN-gamma, nicht an.

Gegenstand der vorliegenden Erfindung sind somit neue in vitro und in vivo zu kultivierende Hybridomzellinien, welche dadurch gekennzeichnet sind, daß diese durch Zellfusion von Milzzellen eines immunisierten Versuchstieres, wobei die erste Immunisierung mit IFN-omega1 oder mit dem Hybridinterferon IFN-omega1/$\alpha$2 und die zweite Immunisierung mit IFN-omega1 erfolgt, mit Myelomzellen erhalten werden und die Antikörper, die die Aktivität von IFN-omega, nicht aber die von IFN-$\alpha$, IFN-$\beta$ oder IFN-$\gamma$ neutralisieren, produzieren sowie

Verfahren zu deren Herstellung, die Verwendung der neuen Antikörper zur Reinigung von IFN-omega und zum Nachweis von IFN-omega, z.B. mittels Immunoassays, in denen diese Antikörper zum spezifischen Nachweis von Humaninterferon vom Typ IFN-omega eingesetzt werden können, wobei zusätzlich auch polyklonale Antikörper eingesetzt werden können.

Erfindungsgemäß wird zur Herstellung der genannten Antikörper wie folgt verfahren:

Die hierfür erforderlichen Antikörper-produzierenden Hybridomzellinien erhält man durch Zellfusion von Milzzellen (siehe Köhler und Milstein in Nature 256, 495-497 (1975)) von entsprechend immunisierten Versuchstieren, z.B. von Mäusemilzzellen, mit Myelomzellen, die bevorzugt selbst keine Antikörper produzieren, z.B. mit Myelomzellen der Linie P3X63Ag8.653 (siehe Kearney et al. in J. Immunol. 123, 1548 (1979)). Dieses Verfahren besteht grundsätzlich darin, daß man eine Maus oder ein anderes geeignetes Tier mit einem Immunogen injiziert. Anschließend werden die von den immunisierten Mäusen, deren Seren Antikörper gegen das injizierte Immunogen enthalten, entnommenen Milzzellen mit Myelomzellen fusioniert. Man erhält Hybridzellen, die als Hybridoma bezeichnet werden, die sich in vitro reproduzieren. Die Population der Hybridoma wird analysiert und manipuliert, so daß man einzelne Klone isoliert, von denen jeder eine einzige, antigen-spezifische Antikörperspezies abscheidet. Jede auf diese Weise erhaltene individuelle Antikörperspezies ist das Produkt einer einzigen B-Zelle aus dem immunisierten Tier, die erzeugt wurde als Reaktion auf eine spezifische immunogene Struktur der immunogenen Substanz. Wird also eine immunogene Substanz in einen lebenden Wirt eingeführt, dann reagiert das Immunsystem des Wirts unter Bildung von Antikörpern gegenüber allen erkennbaren Stellen auf der immunogenen Substanz. Dieser Effekt, nämlich die Bildung von Antikörpern im Abwehrkampf gegen den Eindringling, besteht somit in der Bildung von Antikörpern verschiedener Affinitäten und Spezifitäten für die immunogene Substanz.

Da der Zwei-Stellen-immunometrische Assay auf der Bildung eines Antikörper:Antigen:Antikörper-Sandwich beruht, werden im allgemeinen zwei unterschiedliche monoclonale Antikörper, die sich nicht gegenseitig beim Binden an das Antigen behindern, ausgewählt.

Im vorliegenden Falle werden die Versuchstiere hierzu mit IFN-omega1 oder mit IFN-omega1/$\alpha$2 vorimmunisiert und anschließend nochmals mit IFN-omega1 immunisiert.

Bei der nachfolgenden Zellfusion werden Hybridomkulturen erhalten, welche anschließend einem Screening unterworfen werden, um diejenigen Klone zu identifizieren, die gegen IFN-omega gerichtete Antikörper produzieren. Hierzu werden bevorzugt biologische Tests herangezogen, z.B. Tests die die Neutralisation der biologischen Aktivitäten eines IFN-omega, beispielsweise die der antiviralen Aktivität, durch die hergestellten Antikörper belegen können.

Von den so fünf erhaltenen Kulturen, die als OMG-4, OMG-5, OMG-6, OMG-7 und OMG-8 bezeichnet wurden, und konsistent eine Reduktion der antiviralen Aktivität des IFN-omega1 zeigen, wurden die Klone OMG-4, OMG-5 und OMG-7 zur Antikörperproduktion ausgewählt.

Hierzu können die ausgewählten Hybridomzellinien in vitro oder in vivo kultiviert werden, wobei jedoch die in vivo Kultur bevorzugt ist:

Zellen der ausgewählten Klone werden in mit Pristan oder inkomplettem Freund's Adjuvans vorbehandelte Balb/c Mäuse inokuliert (siehe z.B. Müller et al. in J. Immunol. Methods 87, 193-196 (1986)). Nach 7-18 Tagen wird die Ascites-Flüssigkeit gesammelt und aus dieser mittels Ammoniumsulfat-Fällung und anschließender Affinitätschromatographie oder mit anderen literaturbekannten Methoden der gebildete Antikörper angereichert bzw. isoliert.

Selbstverständlich kann aus einem Zellkulturüberstand einer entsprechenden in vitro-Kultur der gewünschte Antikörper analog isoliert bzw. angereichert werden.

Wie bereits eingangs erwähnt, kann ein so erfindungsgemäß hergestellter neuer Antikörper zur Reinigung und zum Nachweis von IFN-omega, vorzugsweise von IFN-omega1, verwendet werden.

Soll der erfindungsgemäß erhaltenen Antikörper zur Hochreinigung eines IFN-omega verwendet werden, so wird dieser vorzugsweise kovalent an einen biologisch inaktiven Träger gebunden. Die kovalente Bindung des Antikörpers erfolgt hierbei an einen entsprechend aktivierten Träger, vorzugsweise auf Dextran-Basis, z.B. an CNBr-aktivierte Sepharose oder CH-aktivierte Sepharose der Firma Pharmacia, Uppsala. Zur Hochreinigung wird eine Lösung des zu reinigenden omega1-Interferons, welches zweckmäßigerweise entweder gemäß den in der EP-A-0.170.204 beschriebenen Verfahren oder erfindungsgemäß mit Hilfe der der in der EP-A-0.236.920 beschriebenen neuen Plasmide erhalten wird, bei schwach basischen pH, z.B. bei einem pH 7-8, vorzugsweise jedoch bei einem pH 7,5, über einen so hergestellten Antikörper-Affinitätsträger gepumpt, solange bei pH 7,5 gewaschen bis das Eluat proteinfrei ist, und anschließend das gebundene Interferon im sauren Bereich, z.B. mit Hilfe von 0,1 molarer Zitronensäure in 25%igem Äthylenglykol, eluiert. Die so erhaltenen proteinhältigen Fraktionen werden anschließend über einen stark sauren Kationenaustauscher, z.B. den Kationenaustauscher Mono-S der Firma Pharmacia, chromatographiert. Hierbei wird das Humaninterferon des obigen Eluats sofort von der Kationenaustauscher-Säule absorbiert und anschließend mit Hilfe eines NaCl-Gradienten eluiert.

Soll mit Hilfe der neuen Antikörper als Antigen ein omega-Interferon, z.B. IFN-omega1, nachgewiesen bzw. quantitativ bestimmt werden, so stehen hierfür die gebräuchlichen Immunoassaytechniken zur Verfügung.

Die Techniken basieren auf der Bildung eines Komplexes zwischen der zu bestimmenden "antigenen" Substanz und einem oder mehreren Antikörpern, in denen ein Teil oder mehrere Teile des Komplexes markiert sein können, wodurch dann der Nachweis und/oder die quantitative Bestimmung des "Antigens" nach der Abtrennung des komplexen markierten "Antigens" oder Antikörpers ermöglicht wird.

Im Falle einer kompetitiven Immunoassaytechnik liegt die "antigene" Substanz in einer zur untersuchenden Flüssigkeitsprobe im Wettbewerb mit einer bekannten Menge eines markierten "Antigens" für eine limitierte Menge an Antikörperbindungsstellen. Daher ist die Menge des markierten, an den Antikörper gebundenen "Antigens" umgekehrt proportional zu der Menge des "Antigens" in der Probe.

Immunometrische Methoden verwenden demgegenüber markierte Antikörper. Bei einem solchen Assay ist die Menge des mit dem Komplex gebundenen markierten Antikörpers proportional zu der Menge der "antigenen" Substanz, die in der Flüssigkeitsprobe enthalten ist.

Immunometrische Assays sind besonders zum Nachweis von polyvalenten "Antigenen", d.h. "antigenen" Substanzen, die in der Lage sind, mit zwei oder mehr Antikörpern gleichzeitig einen Komplex zu bilden, geeignet. Bei solchen Assays werden typischerweise eine Menge eines unmarkierten Antikörpers, gebunden an einen festen Träger, der in der zu untersuchenden Flüssigkeit unlöslich ist, und eine Menge eines löslichen Antikörpers, der eine Markierung trägt, verwendet, so daß der Nachweis und/oder eine quantitative Bestimmung der Menge des ternären Komplexes, der sich zwischen dem Festphasen-Antikörper, dem "Antigen" und dem markierten Antikörper ausbildet, möglich wird.

Hierzu wird normalerweise der an die feste Phase gebundene Antikörper zunächst mit der zu untersuchenden Probe in Berührung gebracht, um das "Antigen" aus der Probe durch Bildung eines binären Festphasen-Antikörper:Antigen-Komplexes zu extrahieren. Nach einer geeigneten Inkubationsperiode wird der feste Träger gewaschen, um den Rest der Flüssigkeitsprobe, einschließlich nichtumgesetztes "Antigen", falls vorhanden, zu entfernen, und wird dann mit einer Lösung, die eine bekannte Menge des markierten Antikörpers enthält, in Berührung gebracht.

Nach einer zweiten Inkubierungsperiode, bei welcher man den markierten Antikörper einen Komplex mit dem "Antigen" bilden läßt, das an den festen Träger durch den unmarkierten Antikörper gebunden ist, wird der feste Träger ein zweites Mal gewaschen, um nichtumgesetzten markierten Antikörper zu entfernen. In einer einfachen "Ja-Nein"-Assay zur Bestimmung, ob Antigen in der zu untersuchenden Probe vorhanden ist, wird der gewaschene feste Träger untersucht. Die Menge des nachgewiesenen markierten Antikörpers wird mit der einer negativen Kontrollprobe, die frei von dem "Antigen" ist, verglichen. Der Nachweis von markiertem Antikörper in Mengen, die erheblich oberhalb der Hintergrundniveaus sind, wie sie durch eine Negativkontrolle angezeigt werden, gibt dann die Gegenwart des verdächtigen Antigens an. Quantitative Nachweise sind möglich, indem an die Messungen von markierten Antikörpern, die man mit kalibrierten Proben, enthaltend bekannte Mengen des "Antigens", erhält, vergleicht. - Diese Art des Assays wird häufig als ein "Zwei-Stellen"- oder "Sandwich"-Assay bezeichnet, weil das Antigen zwei Antikörper an verschiedenen Stellen seiner Oberfläche gebunden hat.

Bei der vorstehend beschriebenen Assays kommen beispielsweise

als Träger übliche Träger wie Glas, Polystyrol, Polypropylen, Polyethylen, Dextran, Nylon, Amylase, natürliche oder chemisch veränderte Cellulose, Polyacrylamide, Agarose oder Magnetit und

als Marker Enzyme, Radioisotope, Metallchelate oder fluoreszierende, chemilumineszierende und biolumineszierende Verbindungen in Betracht.

Als Enzyme seien beispielsweise Malatdehydrogenase, Staphylococcalnuclease, Delta-5-steroidisomerase, $\alpha$-Glycerin-phosphatdehydrogenase, Triosephosphatisomerase, Meerrettich-Peroxidase, alkalische Phosphatase, Asparaginase, Glukoseoxidase, $\beta$-Galactosidase, Ribonuclease, Urease, Katalase, Glukose-6-phosphatdehydrogenase, Glucoamylase oder Acetylcholinesterase,

als Radioisotope $^3$H, $^{125}$I, $^{127}$I, $^{32}$P, $^{35}$S und $^{14}$C,

als fluoreszierende Verbindungen Fluoreszein-isothiocyanat, Rhodamin, Phycoerythrin, Phycocyanin, Allophycocyanin, o-Phthaldehyd oder Fluorescamin,

als chemilumeszierende Verbindungen Luminol, Isoluminol, ein aromatischer Acridiniumester, Imidazol, ein Acridiniumsalz oder ein Oxalsäureester und

als biolumineszierende Verbindungen Luciferin, Luciferase oder Äquorin genannt.

Desweiteren kann ein erfindungsgemäßer Antikörper mit einem niedermolekularen Hapten wie Biotin, Dinitrophenyl, Pyridoxal oder Fluorescamin verknüpft werden. Diese Haptene können dann durch eine weitere spezifische Reaktion erkannt werden, z.B. Biotin mit Hilfe von Avidin oder Fluorescamin mit Hilfe eines spezifischen Antihapten-Antikörpers.

Desweiteren kann die Aktivität eines als Marker benutzten Enzyms zur Verstärkung des zu messenden Signals verwendet werden.

Besonders bevorzugt ist jedoch die Verwendung von Meerrettich-Peroxidase als Marker, da dieses Enzym mit zahlreichen Substraten reagieren kann. Außerdem ist dieses relativ klein und kann sehr leicht mit einem Antikörper verknüpft werden, beispielsweise mittels der Perjodat-Methode.

Zum Nachweis oder zur quantitativen Bestimmung von einem omega-Interferon, vorzugsweise von IFN-omega1, kommt jedoch vorzugsweise, falls das IFN-omega radioaktiv markiert ist, der kompetitive Radioimmunoassay (RIA), bei welchem insbesondere polyklonale Antikörper bzw. Antikörperseren eingesetzt werden, der immunoradiometrische Assay (IRMA), falls der Antikörper radioaktiv markiert ist, und der "enzym-linked immunosorbent assay" (ELISA), falls der Antikörper mit einem Enzym markiert ist, in Betracht.

Erfindungsgemäß wird IFN-omega, vorzugsweise jedoch IFN-omega1, in einer zu untersuchenden Flüssigkeit nachgewiesen bzw. wie folgt quantitativ bestimmt:

a) Durch Kontaktieren einer zu untersuchenden Probe mit einem Träger, an den ein polyklonaler oder monoklonaler Antikörper gegen das zu bestimmende IFN-omega gebunden ist, und

b) Messung der Bildung des gemäß a) gebildeten binären Komplexes unter Ausbildung eines ternären Komplexes zwischen einem markierten monoklonalen Antikörper und dem gemäß a) gebildeten binären Komplex.

Die zur Durchführung der vorliegenden Erfindung erforderlichen omega-Interferone sind Gegenstand der EP-A-0.170.204 bzw. die nicht in der vorliegenden Erfindung beschriebenen monoklonalen Antikörper, z.B. des Antikörpers OMG-2, sind Gegenstand der EP-A-0.236.920, das Gleiche gilt für die zur Immunisierung verwendeten Hybridinterferone. Die verwendeten polyklonalen Antikörper erhält man nach literaturbekannten Verfahren.

Die folgenden Beispiele sollen die Erfindung näher erläutern, ohne diese jedoch einzuschränken:

Beispiel 1

Herstellung monoklonaler, für IFN-omega spezifischer Antikörper

a) Immunisierung

Eine weibliche, etwa acht Wochen alte Balb/c-Maus wurde mit hochgereinigtem (Reinheit > 95 %) Hybridinterferon IFN-omega1/$\alpha$2 wie folgt immunisiert:

1. Immunisierung:     200 $\mu$g in komplettem Freund's Adjuvans, intraperitoneal
2. Immunisierung:     200 $\mu$g in komplettem Freund's Adjuvans, intraperitoneal, 5 Wochen nach der 1. Immunisierung

Acht Monate nach der zweiten Immunisierung wurde die Maus mit 70 $\mu$g gereinigtem IFN-omega1 (Reinheit > 90 %) weiter immunisiert (inkomplettes Adjuvans, intraperitoneal). Zwölf Tage später wurde eine Serumprobe gewonnen. Neutralisationstests zeigten, daß das Serum der Maus nunmehr relativ hohe Titer von neutralisierenden Antikörpern gegen IFN-omega1 aufwies (vollständige Neutralisation bei bis zu 1000-facher Verdünnung des Serums, partielle Neutralisation bei 10 000-facher Verdünnung). Der Neutralisationstest wurde folgendermaßen durchgeführt: 100 $\mu$l einer Verdünnung der Serumprobe in Zellkulturmedium werden mit 100 $\mu$L einer Lösung von IFN-omega1 (100 antivirale Einheiten/mL) gemischt und 90 Minuten bei 37°C inkubiert. Anschließend wurde die antivirale Aktivität der Proben im biologischen Test (A549 Lungenkarzinom-Zellen, Encephalomyocarditis-Virus) geprüft.

Fünf Wochen nach der dritten Immunisierung wurden der Maus nochmals 70 $\mu$g gereinigtes IFN-omega1 (Reinheit > 90 %) ohne Adjuvans injiziert.

b) Herstellung und Screening von Hybridomen

Die Herstellung von Hybridomen erfolgte nach der ursprünglich von Köhler und Milstein (Nature 256, 495 (1975)) entwickelten Methode unter Verwendung der nicht-sezernierenden Zellinie P3X63Ag8.653 (Kearney et al., J. Immunol. 123, 1548 (1979)). Dazu wurde wie folgt vorgegangen:

Vier Tage nach der letzten Immunisierung (siehe oben) wurde die Milz der Maus entnommen; die Milzzellen wurden mechanisch aus dem Gewebeverband gelöst, in Zellkulturmedium (RPMI 1640 Medium mit Zusatz von Natrium-Penicillin G (100 Einheiten/ml) und Streptomycinsulfat (50 Einheiten/ml)) suspendiert und durch Zentrifugation (Beckmann TJ-6 Zentrifuge, 10 Minuten bei 1000 UpM) gesammelt. 2x10$^8$ Myelomzellen (kultiviert in Zellkulturmedium wie oben mit Zusatz von 10 % fötalem Kälberserum) wurden ebenfalls durch Zentrifugation gesammelt und einmal mit serumfreiem Zellkulturmedium gewaschen. Milzzellen und Myelomzellen wurden schließlich in serumfreiem Zellkulturmedium resuspendiert, die Suspensionen vereinigt und erneut zentrifugiert. Der Überstand wurde entfernt, die Zellen wurden in 3 ml Fusionsmedium (45 % RPMI 1640 Medium, 50 % Polyethylenglykol 4000, 5 % Dimethylsulfoxid) suspendiert und 90 Sekunden vorsichtig geschüttelt, anschließend weitere 60 Sekunden stehengelassen. 3 ml serumfreies Kulturmedium wurden nun tropfenweise über 90 Sekunden zugegeben, die Suspension 60 Sekunden stehengelassen, anschließend weitere 6 ml serumfreies Kulturmedium tropfenweise über 90 Sekunden zugegeben. Schließlich wurden 12 ml Kulturmedium mit 10 % fötalem Kälberserum unter ständigem Rühren langsam zugegeben, 10 Minuten stehengelassen und anschließend auf 50 ml mit Zellkulturmedium mit 10 % fötalem Kälberserum aufgefüllt. Die Zellen wurden durch Zentrifugation gesammelt, in 400 ml Zellkulturmedium mit Zusatz von 20 % fötalem Kälberserum sowie von Hypoxanthin (10$^{-4}$ M), Aminopterin (4x10$^{-7}$ M) und Thymidin (1,6x10$^{-5}$ M), in der Folge HAT-Medium genannt, suspendiert. Zu dieser Suspension wurden weiters peritoneale Macrophagen aus Balb/c Mäusen zugegeben (etwa 50 000/ml); die Suspension wurde schließlich in Zellkulturplatten pipettiert (48 Vertiefungen pro Platte; 0,5 ml pro Vertiefung). Die Platten wurden bei 37°C bebrütet (95 % Luft, 5 % CO$_2$, Wasserdampfsättigung). Nach 3 Tagen wurden zu jeder Kultur 0,5 ml HAT-Medium zugegeben. Von den insgesamt 800 angesetzten Kulturen zeigten nach zwei bis drei Wochen etwa 300 Kulturen Wachstum von Hybridomzellen. Das anschließende Screening wurde folgendermaßen durchgeführt:

Kulturüberstände von mindestens 10-20 % konfluenten Hybridom-Kulturen wurden mit gleichen Volumina einer Lösung von HuIFN-omega1 (20 antivirale Einheiten/mL) gemischt, 90 Minuten bei 37°C inkubiert und anschließend auf ihre antivirale Aktivität getestet. Alle Kulturen wurden in Abständen von jeweils einer Woche mindestens zweifach getestet. Fünf der Kulturen, in der Folge OMG-4, OMG-5, OMG-6, OMG-7 und OMG-8 bezeichnet, zeigten in allen Tests konsistent eine Reduktion der antiviralen Aktivität. Alle Kulturen wurden durch limitierende Verdünnung kloniert, die Klone erneut im beschriebenen Verfahren auf neutralisierende Aktivität getestet. Von jeder Kultur wurden 3 bis 5 positive Klone gepoolt. Zur Antikörperproduktion in vivo wurden von jeder der Hybridomkulturen 3-10x10$^6$ Zellen in Balb/c-Mäusen intraperitoneal inokuliert, in die zwei bis drei Tage zuvor 0,5 ml inkomplettes Freund's Adjuvans oder aber sieben bis zehn Tage zuvor 0,5 ml Pristan intraperitoneal injiziert worden waren. Nach sieben bis 21 Tagen wurde die gebildete Ascites-Flüssigkeit gewonnen; die enthaltenen Antikörper wurden durch Präzipitation mit 50 % Ammoniumsulfat und Affinitätschromatographie über trägergebundenes Protein A nach bekannten Methoden zu über 90 % Reinheit angereichert. Pro ml Ascitesflüssigkeit wurden bei allen Hybridomen etwa 2-5 mg reiner Antikörper erhalten.

c) Charakterisierung der Antikörper OMG-4, OMG-5 und OMG-7

Alle Antikörper zeigten in der Natriumdodecylsulfat-Polyacrylamid-Elektrophoreseunter nicht-reduzierenden Bedingungen sowie in der Gelpermeations-Hochdruckflüssigkeitschromatographie ein mit einem IgG-Markerprotein identisches Retentionsverhalten und sind somit vom IgG-Typ. In einem Neutralisationstest, in dem die Hemmung der antiviralen Wirkung von Interferonen geprüft wurde (siehe oben), neutralisierten alle Antikörper bei einer Konzentration von 100 $\mu$g/mL die Aktivität von IFN-omega1, nicht aber die von IFN-$\alpha$2c, IFN-$\beta$ oder IFN-gamma. Diese 3 Klone wurden gemäß Budapester Vertrag bei der European Collection of Animal Cell Cultures, PHLS Centre for Applied Microbiology and Research, Porton Down, Salisbury, Wiltshire SP4 OJG, United Kingdom, am 14. August 1987 unter den ECACC-Nummern 87081401 (OMG-4), 87081402 (OMG-5) und 87081403 (OMG-7) hinterlegt.

Beispiel 2

Enzymimmunoassays (ELISA) für IFN-omega1

Die Antikörper OMG-5 und OMG-7 wurden nach bekannten Methoden (siehe zum Beispiel Wilson M.B. und Nakane P.K., in Immunofluorescence and Related Staining Techniques, Herausgeber W. Knapp et al., S. 215-224; Elsevier 1978) kovalent an Meerrettich-Peroxidase gebunden. Dazu wurde wie folgt verfahren:
2 mg Meerrettich-Peroxidase in Wasser wurden mit 0,2 ml 100 mM Natriumperjodat versetzt und 40 Minuten bei Raumtemperatur geschüttelt, gegen 2 x 500 ml 1 mM Natrium-Acetat pH 4,4 über Nacht bei 4°C dialysiert; anschließend wurde die Lösung mit 0,1 M NaHCO$_3$ pH 9,5 auf einen pH von etwa 9 eingestellt. Zu dieser Lösung wurde eine Lösung des monoklonalen Antikörpers (OMG-5, 2 ml mit 1,6 mg/ml oder OMG-7; 1,5 ml mit 4,7 mg/ml, jeweils in 10 mM NaHCO$_3$ pH 9,5) zugegeben und zwei Stunden bei Raumtemperatur geschüttelt. 100 $\mu$l einer Lösung von NaBH$_4$ (4 mg/ml in Wasser) wurden zugegeben und die Lösung weitere zwei Stunden im Eisbad inkubiert; anschließend wurden 3 ml kalte gesättigte Ammonsulfatlösung zugetropft und eine Stunde im Eisbad inkubiert. Das entstandene Präzipitat von Peroxidase-Immunglobulin-Konjugat wurde durch Zentrifugation gesammelt, in 1 ml phosphat-gepuffertes isotoner Kochsalzlösung pH 7,4 gelöst und durch Zugabe von 1 ml einer Lösung von Rinderserumalbumin (10 mg/ml in phosphat-gepufferter Kochsalzlösung) stabilisiert. Die Lösung wurde bei -70°C eingefroren.
Festphasen-Sandwich-Enzymimmunoassays für IFN-omega1 wurden nach im allgemeinen bekannten Verfahren durchgeführt (siehe zum Beispiel Berthold, W., Merk, W. und Adolf, G.R., Arzneim.-Forschung./Drug Res. 35, 364-369 (1985)). Dabei wurden zur Beschichtung der Mikrotiter-ELISA-Testplatten die monoclonalen Antikörper OMG-2, OMG-5 oder OMG-7 in einer Konzentration von 10 $\mu$g/ml in 0.1 M Natriumkarbonat pH 9.5 eingesetzt (100 $\mu$l pro Vertiefung) und die Platten entweder eine Stunde bei Raumtemperatur oder über Nacht bei 4-8°C inkubiert. Die Antikörperlösung wurde entfernt, die Vertiefungen mit jeweils 200 $\mu$l Wasser gewaschen und mit 100 $\mu$l einer Lösung von Rinderserumalbumin (5 mg/ml) in phosphatgepufferter, isotoner Kochsalzlösung pH 7,4 (in der Folge PBS/BSA genannt) befüllt. Anschließend wurden jeweils 100 $\mu$l einer Lösung von IFN-omega1 in einer Konzentration von 20 ng/ml zugegeben, durchmischt und durch seriellen Transfer von jeweils 100 $\mu$l eine Verdünnungsreihe hergestellt. Schließlich wurden zu allen Vertiefungen 50 $\mu$l einer Lösung des Antikörper-Enzym-Konjugats(OMG-5/Peroxidase oder OMG-7/Peroxidase, Stammlösung (siehe oben) 1:10 000 in PBS/BSA verdünnt) zugegeben und die Platten 3 Stunden bei Raumtemperatur inkubiert. Anschließend wurde die Lösung entfernt, die Vertiefungen dreimal mit Wasser gewaschen und mit jeweils 100 $\mu$l Substratlösung (ortho-Phenylendiamin, 3 mg/ml und Natriumperborat, 1 mg/ml in 0,067 M Kaliumzitrat pH 5) gefüllt. Nach 30 Minuten Inkubation bei Raumtemperatur wurden zu jeder Vertiefung 100 $\mu$l 4 N Schwefelsäure pipettiert; anschließend wurde die optische Dichte bei 492 nm in einem Mehrkanal-Photometer (ELISA-Lesegerät) gemessen.

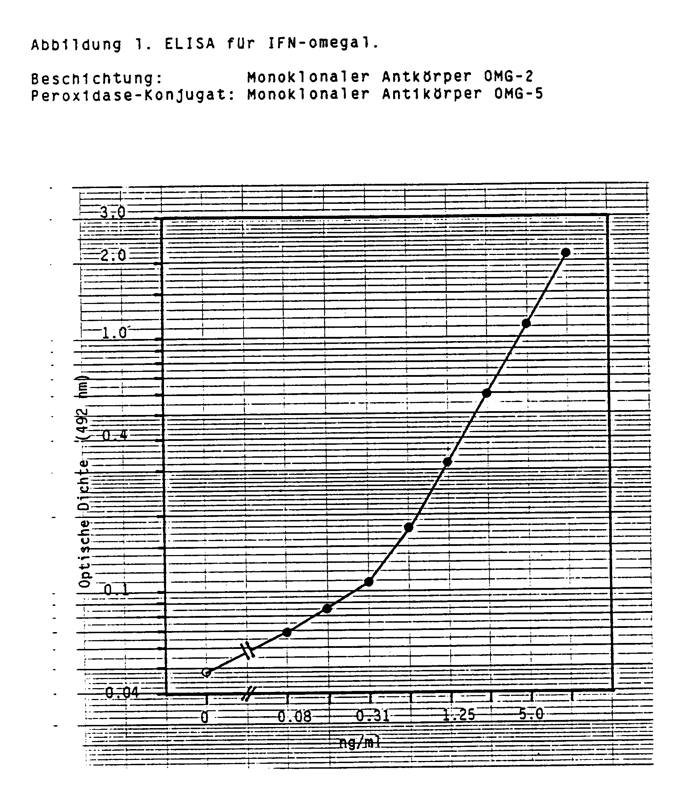

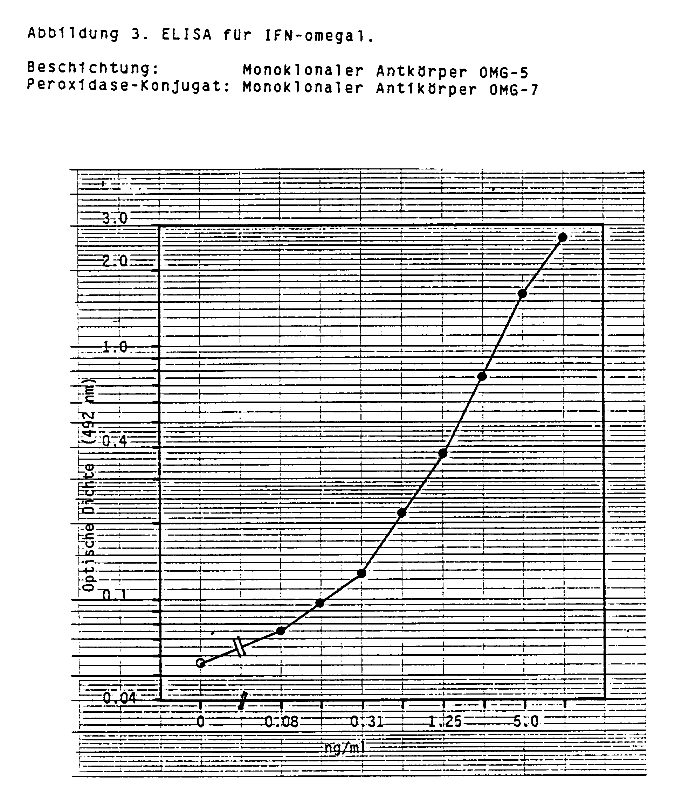

Mit allen heterologen Kombinationen von Beschichtungs-Antikörper und Antikörper-Peroxidase-Konjugat wurden dosisabhängige Absorptionsänderungen erzielt. Die Abbildungen 1, 2 und 3 zeigen die erhaltenen Kurven.

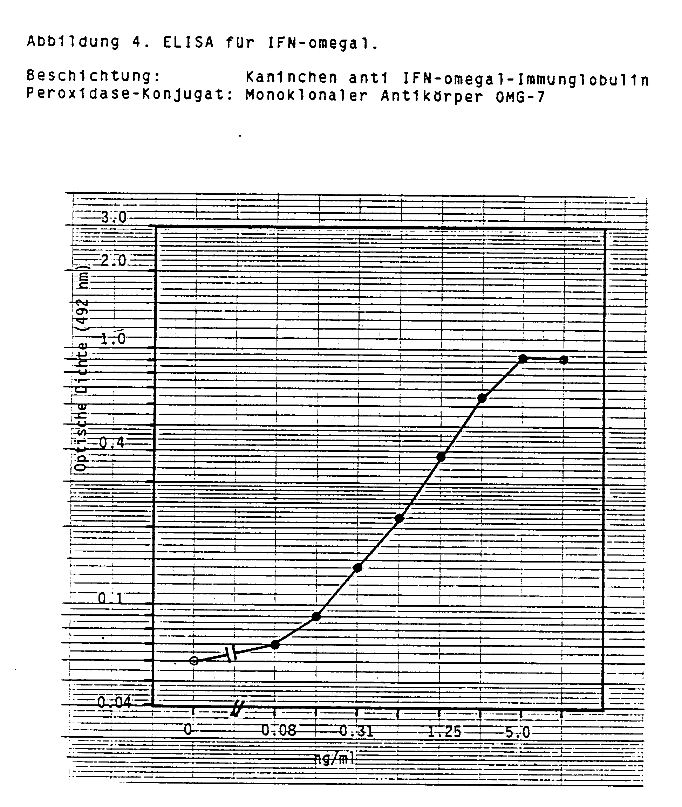

Zur Beschichtung konnte auch ein Kaninchen-anti-IFN-omega1 Immunoglobulin, gewonnen durch zweimalige Immunisierung eines Kaninchens mit IFN-omega1 und partielle Reinigung aus dem Serum durch Präzipitation mit 50 % Ammoniumsulfat, in einer Konzentration von 10 $\mu$g/ml eingesetzt werden (siehe Abbildung 4).

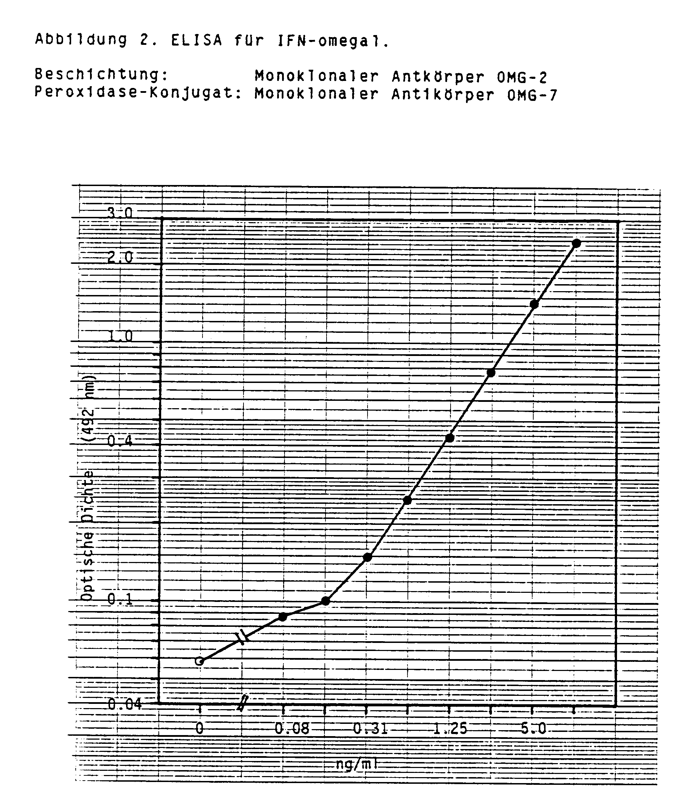

Die Spezifität des aus dem Antikörper OMG-2 (siehe EP-A-0.236.920) und Peroxidase-gebundenem Antikörper OMG-7 aufgebauten ELISA (siehe Abbildung 2) für IFN-omega wurde geprüft, indem Präparate anderer humaner Interferone in einem sehr weiten Konzentrationsbereich aufgetragen wurden. Folgende Interferone wurden dabei eingesetzt:

| Interferon | Quelle | Konzentrationsbereich |
|---|---|---|
| IFN-$\alpha$1 | rekombinant (E.coli) | 2 ng - 50 $\mu$g/ml |
| IFN-$\alpha$2c | rekombinant (E.coli) | 2 ng - 50 $\mu$g/ml |
| IFN-$\alpha$B | rekombinant (E.coli) | $3x10^2-1,25x10^6$ E/ml |
| IFN-$\alpha$F | rekombinant (E.coli) | $1,4x10^1-3,50x10^5$ E/ml |
| IFN-$\beta$ | Fibroblasten, mit Poly (I:C) induziert | $8x10^2-2x10^6$ E/ml |
| IFN-gamma | rekombinant (E.coli) | 2 ng - 50 $\mu$g/ml |

Dabei wurde bei einer Sensitivität von 100 pg/ml für IFN-omega1 mit keinem der Präparate bei keiner Konzentration ein signifikantes Signal beobachtet. Der ELISA kann daher nicht nur zur Quantifizierung von rekombinantem IFN-omega1, sondern auch beispielsweise zur Bestimmung des Anteils an IFN-omega1 in Leukocyteninterferon oder anderen aus Zellkulturen gewonnenen Interferon-Präparaten verwendet werden.

Beispiel 3

Immunradiometrischer Assay (IRMA) für IFN-omega1

Der Antikörper OMG-7 wurde nach an sich bekannter Methode mit Hilfe von N-Succinimidyl[2,3-$^3$H]-propionat ($^3$H-NSP, Firma Amersham International, England; 110 Ci/mMol) radioaktiv markiert. Dazu wurde in einem silikonisierten Probengefäß 1 mCi der Lösung von $^3$H-NSP im Vakuum zur Trockene gebracht. Anschließend wurden 50 $\mu$g einer Lösung des monoklonalen Antikörpers OMG-7 (4,7 mg/ml) in gepufferter Kochsalzlösung pH 7,4 zupipettiert und 3 $\mu$l 1 M Boratpuffer pH 8,5 zugegeben. Nach 24 Stunden bei 4°C wurde das überschüssige $^3$H-NSP mit 20 $\mu$l 1 M Glycin in Boratpuffer abgefangen, mit 250 $\mu$l 50 mM Kaliumphosphat-Puffer pH 7,4, mit 150 mM NaCl und 5 mg/ml Rinderserumalbumin verdünnt und über eine Sephadex G 50 M - Säule (0,5 x 20 cm) vom markierten Antikörper abgetrennt. Der Antikörper zeigte eine spezifische Aktivität von etwa 10 Ci/g Protein.

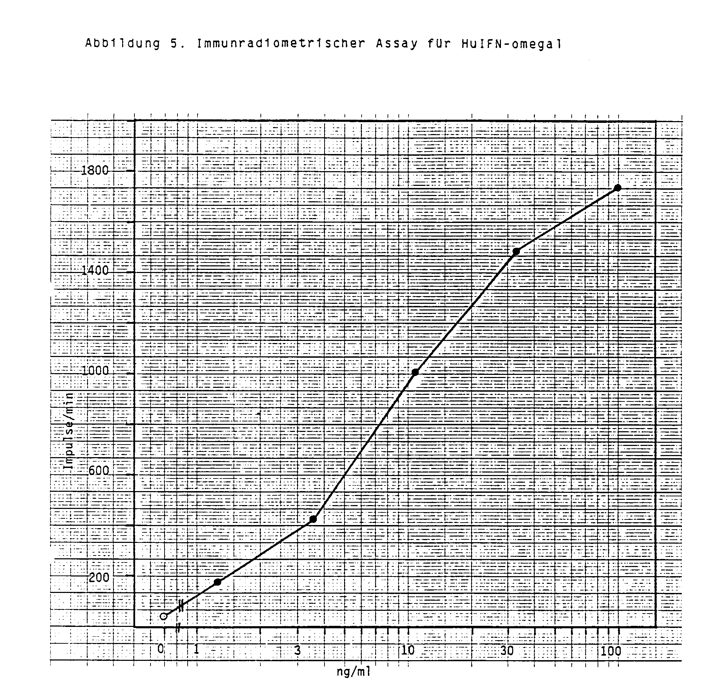

Zur Durchführung des Tests wurden geätzte Polystyrol-Kügelchen (Durchmesser 6,5 mm; Firma Northumbria Biologicals, England) mit dem Antikörper OMG-2 beschichtet (10 $\mu$g/ml in 0,1 M Natriumkarbonat pH 9,5; 1 Stunde bei Raumtemperatur). Die Kügelchen wurden in PBS/BSA (siehe Beispiel 2) eine Stunde inkubiert und danach zweimal mit je 250 $\mu$l Wasser gewaschen. Die Kügelchen wurde in geeigneten Eprouvetten mit jeweils 200 $\mu$l einer Lösung von IFN-omega1 in steigenden Konzentrationen in PBS/BSA 3 Stunden bei 4°C inkubiert und dreimal mit 250 $\mu$l Wasser gewaschen. Anschließend wurden je 200 $\mu$l einer Lösung des markierten Antikörpers (100 ng/ml in PBS/BSA; pro Röhrchen ca. 27000 Impulse/Minute) zugegeben und 20 Stunden bei 4°C inkubiert. Danach wurden die Kügelchen dreimal mit 20 $\mu$l Wasser gewaschen, in Polypropylen-Röhrchen transferiert und die gebundene Radioaktivität nach Zugabe von 4 ml Szintillator-Cocktail im Flüssigkeitsszintillationszähler gemessen. Abbildung 5 zeigt die gebundene Radioaktivität in Abhängigkeit von der Interferonkonzentration.

**Patentansprüche**

1. In vitro und in vivo zu kultivierende Hybridomzellinien, dadurch gekennzeichnet, daß diese durch Zellfusion von Milzzellen eines immunisierten Versuchstieres, wobei die erste Immunisierung mit IFN-omega1 oder mit dem Hybridinterferon IFN-omega1/$\alpha$2 und die zweite Immunisierung mit IFN-omega1 erfolgt, mit Myelomzellen erhalten werden und Antikörper, die die Aktivität von IFN-omega, nicht aber

die von IFN-$\alpha$, IFN-$\beta$ oder IFN-$\gamma$ neutralisieren, produzieren.

2. Hybridomzellinien gemäß Anspruch 1, dadurch gekennzeichnet, daß als Milzzellen die von Balb/c Mäusen und als Myelomzellen die der Linie P3X63Ag8.653 verwendet werden.

3. Hybridomzellinien gemäß den Ansprüchen 1 und 2 bezeichnet als OMG-4 (hinterlegt unter der ECACC-Nummer 87081401), OMG-5 (hinterlegt unter der ECACC-Nummer 87081402) und OMG-7 (hinterlegt unter der ECACC-Nummer 87081403).

4. Monoklonale Antikörper vom IgG-Typ, die die Aktivität eines omega-Interferons neutralisieren, aber nicht die des IFN-$\beta$, IFN-$\gamma$ oder eines $\alpha$-Interferons, erhältlich durch Kultivierung einer Hybridomzellinie gemäß den Ansprüchen 1 bis 3.

5. Monoklonale Antikörper gemäß Anspruch 4, dadurch gekennzeichnet, daß diese durch die Klone OMG-4, OMG-5 und OMG-7, welche unter den ECACC-Nummern 87081401, 87081402 und 87081403 hinterlegt sind, gebildet werden und bei einer Konzentration von 100 $\mu$g/mL die Aktivität von IFN-omega1, nicht aber die von IFN-$\alpha$2c, IFN-$\beta$ oder IFN-$\gamma$ neutralisieren.

6. Monoklonale Antikörper gemäß den Ansprüchen 4 oder 5, dadurch gekennzeichnet, daß diese markiert sind.

7. Verfahren zur Herstellung der monoklonalen Antikörper gemäß den Ansprüchen 4 bis 6, dadurch gekennzeichnet, daß nach Wachstum der Hybridomzellinien gemäß den Ansprüchen 1 bis 3 die IgG-Antikörper mit Anti-omega-Interferon-Spezifität isoliert werden und gegebenenfalls ein so erhaltener monoklonaler Antikörper mit einem Marker verbunden wird.

8. Verwendung der neuen monoklonalen Antikörper gemäß den Ansprüchen 4 oder 5 zur Reinigung der omega-Interferone.

9. Antikörper-Affinitätsträger geeignet zur Reinigung der omega-Interferone, dadurch gekennzeichnet, daß ein monoklonaler Antikörper gemäß den Ansprüchen 4 oder 5 kovalent an einen geeigneten aktivierten Träger gebunden wird.

10. Verfahren zur Herstellung eines Antikörper-Affinitätsträgers gemäß Anspruch 9, dadurch gekennzeichnet, daß ein monoklonaler Antikörper gemäß den Ansprüchen 4 oder 5 kovalent an einen geeigneten aktivierten Träger kovalenten gebunden wird.

11. Verfahren zur Reinigung von einem omega-Interferon, dadurch gekennzeichnet, daß IFN-omega an einen Antikörper-Affinitätsträger gemäß Anspruch 9 gebunden wird und anschließend wieder eluiert wird.

12. Verwendung der neuen monoklonalen Antikörper 4 oder 5 und deren markierte Derivate gemäß Anspruch 6 zum Nachweis von IFN-omega.

13. Ein zum Nachweis von IFN-omega geeigneter Kit, dadurch gekennzeichnet, daß dieser einen monoklonalen Antikörper gemäß den Ansprüchen 4 bis 6 enthält.

14. Verfahren zum Nachweis von IFN-omega, dadurch gekennzeichnet, daß eine Probe mit einem Antikörper gemäß Anspruch 6 inkubiert wird und die Reaktion des Antikörpers mit IFN-omega registriert wird.

15. Ein zum Nachweis oder zur quantitativen Bestimmung von IFN-omega geeigneter Kit, dadurch gekennzeichnet, daß dieser eine feste Phase enthält, an welche
   a) ein monoklonaler oder polyklonaler Antikörper gegen IFN-omega gebunden ist und
   b) der gebildete binäre Komplex mittels eines monoklonalen Antikörpers gemäß Anspruch 6 bestimmt wird.

16. Verfahren zum Nachweis oder zur quantitativen Bestimmung von IFN-omega, dadurch gekennzeichnet, daß eine Probe

a) mit einem monoklonalen oder polyklonalen Antikörper gegen IFN-omega, der an einen Träger gebunden ist, inkubiert wird,

b) die so erhaltene Probe mit einem Antikörper gemäß Anspruch 6 versetzt wird und

c) der Gehalt des so gebundenen Antikörpers gemäß Anspruch 6 bestimmt wird.

17. Immunoassay gemäß den Ansprüchen 13 oder 15, dadurch gekennzeichnet, daß dieser nur IFN-omega1, nicht aber IFN vom Typ $\alpha$, $\beta$ oder $\gamma$ erkennt.

**Claims**

1. Hybridoma cell lines for cultivation in vitro and in vivo, characterised in that these are obtained by cell fusion of spleen cells from an immunised experimental animal, immunised first with IFN-omega1 or a hybrid interferon IFN-omega1/$\alpha$2, and secondly with IFN-omega1, with myeloma cells and that they produce antibodies which neutralise the activity of IFN-omega, but not that of IFN-$\alpha$, IFN-$\beta$ or IFN-$\gamma$.

2. Hybridoma cell lines as claimed in claims 1 and 2, characterised in that the spleen cells used are those from Balb/c mice, and the myeloma cells used are of the line P3X63Ag8.653.

3. Hybridoma cell lines as claimed in claims 1 and 2 designated as OMG-4 (deposited under ECACC-No. 87 081401), OMG-5 (deposited under ECACC-No. 87 081402) and OMG-7 (deposited under EcACC-No. 87 081403).

4. Monoclonal antibodies of the IgG type, characterised in that they neutralise the activity of an omega interferon but do not neutralise the activity of IFN-$\beta$, IFN-$\gamma$ or $\alpha$-interferons, obtainable by cultivating a hybridoma cell line according to claims 1 to 3.

5. Monoclonal antibodies as claimed in claim 4, characterised in that these are formed by the clones OMG-4, OMG-5 and OMG-7, which are deposited under ECACC Nos. 87 081401, 87 081402 and 87 081403, and at a concentration of 100 $\mu$g/mL, they neutralise the activity of IFN-omega1 but not that of IFN-$\alpha$2c, IFN-$\beta$ or IFN-$\gamma$.

6. Monoclonal antibodies as claimed in claim 4 or 5, characterised in that they are labelled.

7. Process for preparing the monoclonal antibodies as claimed in claims 4 to 6, characterised in that, after the growth of the hybridoma cell lines according to claims 1 to 3, the IgG antibodies with anti-omega-interferon specificity are isolated and if desired a marker is bound to a thus obtained monoclonal antibody.

8. Use of the new monoclonal antibodies as claimed in claim 4 or 5 for the purification of omega interferons.

9. Antibody affinity carrier suitable for purifying omega interferons, characterised in that a monoclonal antibody as claimed in claim 4 or 5 is covalently bound to a suitable activated carrier.

10. Process for preparing an antibody affinity carrier as claimed in claim 9, characterised in that a monoclonal antibody as claimed in claim 4 or 5 is covalently bound to a suitable activated carrier.

11. Process for purifying an omega interferon, characterised in that IFN-omega is bound to an antibody-affinity carrier as claimed in claim 9 and then reeluted.

12. Use of the new monoclonal antibodies as claimed in claim 4 or 5 and the labelled derivatives thereof as claimed in claim 6 for detecting IFN-omega.

13. A kit suitable for the detection of IFN-omega, characterised in that it contains a monoclonal antibody as claimed in claims 4 to 6.

14. Process for detecting IFN-omega, characterised in that a sample is incubated with an antibody as claimed in claim 6 and the reaction of the antibody with IFN-omega is recorded.

**15.** A kit suitable for the detection or quantitative determination of IFN-omega, characterised in that it contains a solid phase to which

a) a monoclonal or polyclonal antibody against IFN-omega is bound and

b) the binary complex formed is determined by means of a monoclonal antibody as claimed in claim 6.

**16.** Process for detecting or quantitatively determining IFN-omega, characterised in that a sample

a) is incubated with a monoclonal or polyclonal antibody against IFN-omega which is bound to a carrier,

b) the sample thus obtained is mixed with an antibody as claimed in claim 6 and

c) the amount of the antibody as claimed in claim 6 thus bound is determined.

**17.** Immunoassay as claimed in claim 13 or 15, characterised in that it recognises only IFN-omega1 but not IFN of the $\alpha$, $\beta$ or $\gamma$ type.

**Revendications**

**1.** Lignées cellulaires d'hybridome à cultiver in vitro et in vivo, caractérisées en ce qu'elles sont obtenues par fusion cellulaire de cellules spléniques d'un animal d'expérience immunisé, la première immunisation étant réalisée avec IFN-oméga1 ou avec l'interféron hybride IFN-oméga1/$\alpha$2et la deuxième immunisation étant réalisée avec IFN-oméga1, avec des cellules de myélome et produisent des anticorps qui neutralisent l'activité de IFN-oméga mais pas celle de IFN-$\alpha$, IFN-$\beta$ ou IL-$\gamma$.

**2.** Lignées cellulaires d'hybridome selon la revendication 1, caractérisées en ce que l'on utilise comme cellules spléniques celles de souris Balb/c et comme cellules de myélome celles de la lignée P3X63Ag8.653.

**3.** Lignées cellulaires d'hybridome selon les revendications 1 et 2, appelées OMG-4 (déposée sous le numéro ECACC 87081401), OMG-5 (déposée sous le numéro ECACC 87081402) et OMG-7 (déposée sous le numéro ECACC 87081403).

**4.** Anticorps monoclonaux de type IgG, qui neutralisent l'activité d'un interféron oméga mais pas celle de IFN-$\beta$, IFN-$\gamma$ ou d'un interféron $\alpha$, et qui peuvent être obtenus par culture d'une lignée cellulaire d'hybridome selon les revendications 1 à 3.

**5.** Anticorps monoclonaux selon la revendication 4, caractérisés en ce qu'ils sont formés par les clones OMG-4, OMG-5 et OMG-7, qui sont déposés sous les numéros ECACC 87081401, 87081402 et 87081403, et neutralisent à une concentration de 100 $\mu$g/ml l'activité de IFN-oméga1, mais pas celle de IFN-$\alpha$2c, IFN-$\beta$ ou IFN-$\gamma$.

**6.** Anticorps monoclonaux selon la revendication 4 ou 5, caractérisés en ce qu'ils sont marqués.

**7.** Procédé de préparation des anticorps monoclonaux selon les revendications 4 à 6, caractérisé en ce qu'après la croissance des lignées cellulaires d'hybridome selon les revendications 1 à 3, les anticorps IgG à spécificité antiinterféron oméga sont isolés et éventuellement un anticorps monoclonal ainsi obtenu est lié à un marqueur.

**8.** Utilisation des nouveaux anticorps monoclonaux selon la revendication 4 ou 5 pour la purification des interférons oméga.

**9.** Support d'anticorps à affinité convenant pour la purification des interférons oméga, caractérisé en ce qu'un anticorps monoclonal selon la revendication 4 ou 5 est lié de manière covalente à un support activé approprié.

**10.** Procédé de préparation d'un support d'anticorps à affinité selon la revendication 9, caractérisé en ce qu'un anticorps monoclonal selon la revendication 4 ou 5 est lié de manière covalente à un support activé approprié.

**11.** Procédé de purification d'un interféron oméga, caractérisé en ce que IFN-oméga est lié à un support d'affinité d'anticorps selon la revendication 9 puis est de nouveau élué.

**12.** Utilisation des nouveaux anticorps monoclonaux selon la revendication 4 ou 5 et de leurs dérivés marqués selon la revendication 6 pour la mise en évidence de IFN-oméga.

**13.** Trousse convenant pour la mise en évidence de IFN-oméga, caractérisée en ce qu'elle contient un anticorps monoclonal selon les revendications 4 à 6.

**14.** Procédé de mise en évidence de IFN-oméga, caractérisé en ce qu'un échantillon est incubé avec un anticorps selon la revendication 6 et la réaction de l'anticorps avec IFN-oméga est enregistrée.

**15.** Trousse convenant pour la mise en évidence ou pour la détermination quantitative de IFN-oméga, caractérisée en ce qu'elle contient une phase solide à laquelle
   a) un anticorps monoclonal ou polyclonal contre IFN-oméga est lié et
   b) le complexe binaire formé est déterminé au moyen d'un anticorps monoclonal selon la revendication 6.

**16.** Procédé de mise en évidence ou de détermination quantitative de IFN-oméga, caractérisé en ce qu'un échantillon
   a) est incubé avec un anticorps monoclonal ou polyclonal contre IFN-oméga qui est lié à un support,
   b) l'échantillon ainsi obtenu est mis à réagir avec un anticorps selon la revendication 6 et
   c) la teneur en anticorps selon la revendication 6 ainsi lié est déterminée.

**17.** Test immunologique selon la revendication 13 ou 15, caractérisé en ce qu'il reconnait seulement IFN-oméga1 mais pas IFN de type $\alpha$, $\beta$ ou $\gamma$.

Abbildung 1. ELISA für IFN-omega1.

Beschichtung:          Monoklonaler Antkörper OMG-2
Peroxidase-Konjugat: Monoklonaler Antikörper OMG-5

Abbildung 2. ELISA für IFN-omega1.

Beschichtung:          Monoklonaler Antkörper OMG-2
Peroxidase-Konjugat: Monoklonaler Antikörper OMG-7

Abbildung 3. ELISA für IFN-omega1.

Beschichtung:                Monoklonaler Antkörper OMG-5
Peroxidase-Konjugat: Monoklonaler Antikörper OMG-7

Abbildung 4. ELISA für IFN-omega1.

Beschichtung:          Kaninchen anti IFN-omega1-Immunglobulin
Peroxidase-Konjugat: Monoklonaler Antikörper OMG-7

Abbildung 5. Immunradiometrischer Assay für HuIFN-omega1